# EUROPEAN PATENT SPECIFICATION

(11) **EP 0 594 628 B1**
(45) Date of publication and mention of the grant of the patent: **29.12.1999**
(21) Application number: 92904586.2
(22) Date of filing: 04.02.1992
(51) Int. Cl.: A61L 15/36, A61F 13/15

(54) **TAMPON OR SANITARY NAPKIN PROVIDED WITH LACTIC ACID PRODUCING BACTERIA**
TAMPON ODER MONATSBINDE MIT MILCHSÄUREBAKTERIEN
TAMPON OU SERVIETTE SANITAIRE COMPORTANT DES BACTERIES PRODUISANT DE L'ACIDE LACTIQUE

(30) Priority: 05.02.1991 SE 9100364
(43) Date of publication of application: 04.05.1994
(73) Proprietor: LECUR DEVELOPMENT IN SWEDEN AKTIEBOLAG, S-262 73 Angelholm (SE)
(72) Inventor: KVANTA, Endre, S-262 61 Ängelholm (SE)
(74) Representative: Roth, Ernst Adolf Michael
(86) International application number: SE9200061
(87) International publication number: WO9213577

(56) References cited:
- EP-A- 0 130 356
- DE-A- 2 309 575
- GB-A- 2 107 192
- SE-A- 8 505 491

## Description

The present invention relates to the production and use of a tampon or sanitary napkin being impregnated with a culture of living lactic acid producing bacteria, preferably isolated from the genital region of healthy women with the object of alleviate or vaginal or urinary tract infections, alleviate or counteract the growth of undesired micro-organisms, alleviate or counteract vaginal discharge, the formation of odour producing secretion, as well as alleviate or counteract hygienic complaints caused by these as well as contribute to a more rapid recovery of the natural flora of lactic acid producing bacteria of the urogenital region after an antibiotic treatment.

It is known that the urogenital region consists of a rich flora of micro organisms, preferably bacteria and fungi. It is also known that lactic acid producing bacteria of the Lactobacillus group dominates the micro flora of healthy women both prior to and after menopause and the most of these lactobacillus have an ability to sustain the growth and reduce the patogenicity of many uropathogens. The mechanism behind the antagonistic effect is not completely derived but the dominating comprehension is that lactobacillus having antagonistic properties have an ability of coaggregate with the uropathogens, to produce inhibitors and to lower pH in the urogenital environment by the lactic acid production.

In connection with an infection of the urogenital region, UTI (Urogenital Tract Infection) a large increase of the number of uropathogens can be observed in the vagina and/or the urinary tract compared with healthy individuals.

It is also known that the antagonistic properties of lactobacillus and other lactic acid producing bacteria against pathogens is partly denoted their ability of producing different so called antimetabolites, such as lactic acid, hydrogen peroxide, bacteriocins, etc. One has also described how bacteriocins from lactobacillus seem to function against pathogens as bacteria and fungi.

It is further known that one has tried to explore the antagonistic properties of the lactobacillus with the object of preventing urogenital infections. The literature describes e.g. that suspensions as well as suppositories comprising living lactobacillus have been used for this purpose. The same process has also been used for the purpose of being able to restore a natural vaginal flora more rapidly after a treatment of the urogenital region using antibiotics.

It is also known that living lactobacillus packed in gelatine capsules have been used as well for that purpose. Such products are available now and then on the market, as well as suppositories.

The problem of urogenital infections is great among women both prior to and after menopause. The troubles are widely spread. According to the literature up to 25% of all women will get an UTI some time during their lifetime, and more than 80% of these get repeated recidive. Urogenital infections not only cause a great suffering of those afflicted but cause also great economical consequences for the individual as well as for the public.

UTI often requires long term antibiotic treatment. Up to two years of treatment using antibiotics is known from the literature. Most UTI can however, be cured after some months of antibiotic treatment. However, it is so that there are few possibilities to cure UTI if the patient is not willing to undergo a long term antibiotic treatment, due to the negative side effects which can be caused by the antibiotic therapy. As the use of antibiotic for prophylactic purpose not can be recommended and that the above methods to use viable lactobacillus either are regarded as nonhygienic, unesthetical or provide a small or no effect at all, the need is great to be able to use a prophylactic tool against urogenital infections in a cheap, simple and rational way.

It has turned out that this desire and the present problem can be meet by best by means of the present invention, which is characterized in that a tampon or sanitary napkin is impregnated with viable cultures of lactic acid producing bacteria particularly of the genus Pediococcus. The lactic acid producing bacteria to be used for this purpose shall show the following criteria. They shall preferably be naturally occurring in the urogenital region of heathy individuals, they shall have antagonistic properties against uropathogens, they shall be stable at cultivation and other technical handling, (i.e. they shall maintain their genetic properties in connection with or after a industrial production handling) and they shall be able to be administered in a lyophilized form.

The bacteria organisms that best meet these criteria belongs to the genus Pediococcus.

Comparative investigations made have also shown that bacteria belonging to the Lactobacillus group do not fulfils all these requirements. The primary reason to the uncertainty using Lactobacillus as an antagonistic micro-organism at UTI or against UTI is their well-known instability at a technical handling thereof. It has turned out that lactobacillus per se provides an acceptable protection against urogenital infections as long as one uses the natural flora of Lactobacillus present of the respective individual. The desired antagonistic properties of the Lactobacillus, their genetic stability, the viability etc are, however, lost to a greater or less extent when they have been isolated from the naturally occurring environment and in connection with the subsequent technical handling thereof. The genetic instability of the Lactobacillus at technical handling is well-known and well documented. The lactobacillus isolated from the genital tract or from the natural human intestinal tract are hereby no exception.

Contrary to this it has turned out that pediococcus isolated from the genital tract keep stable during and after a necessary technical treatment, i.e. they maintain their genetic identity, their lactic acid producing ability, viability as well as the valuable antagonistic properties against uropathogens even after a long term storing.

By using pediococcus as a main organism at the impregnation of tampons or sanitary napkins according to the present invention we have succeeded in eliminating all practical problems which have inhibited a successful use of the previously proposed Lactobacillus.

As a further essential advantage using the present invention it can be mentioned that compared with lactobacillus the pediococcus are easily cultivated, have a rapid growth, high yield, and are readily handled at technical handling thereof. These circumstances make the pediococcus also cheaper than the lactobacillus at the proposed use according to the present invention.

Impregnation of the tampon or sanitary napkin using freeze-fried viable lactic acid producing bacteria will be illustrated in the following non-restricting examples.

The term sanitary napkin shall mean sanitary napkin as well as any other means applied topically against the outer genitalia.

A tampon or sanitary napkin is coated with any compound having adhesive properties , such as coco fat, vegetabilic oil, or other glyceride or wax or any other compound having the similar properties and a good skin and mucous membrane tolerance at the use in the urogenital region. A culture of viable, freeze-dried bacteria is added then in such a way that the bacteria attach on the coated area. The impregnated layer is then protected with a permeable layer of a suitable material, e.g. the same material as having been used for the production of the tampon of sanitary napkin. When the tampon or sanitary napkin then become wet at the use thereof the bacteria become rehydrated in the fluid added and by natural diffusion they will thus migrate and establish in the urogenital region. It is also known that viable, freeze-dried lactic acid producing bacteria starts a logarithmic growth phase after some revitalization time.

Practical tests have shown that the use of a tampon or sanitary napkin impregnated with living viable lactic acid producing bacteria having the above given properties provides for a simple and efficient protection against UTI. It has also turned out that the same type of tampon or sanitary napkin can preferably be used not only prior to but even after menopause. It has also turned out that a tampon or sanitary napkin of the present invention can be used with a good result to rapidly restore the natural flora of micro-organisms of the urogenital region after an antibiotic treatment. It has also turned out to be important to use the right composition of the carrier of the lactic acid producing bacteria. As carrier of the freeze-dried lactic acid producing bacteria carbohydrates are suitably used which the bacteria can use for production of lactic acid, and essential growth promoting agents which facilitates the establishment of the urogenital region. It has thereby turned out that some B-vitamins are such growth promoting agents.

Lactic acid producing bacteria that are suitably used for this purpose are isolated from the genital region of healthy women and belong to the genera Pediococcus, Lactobacillus, but also Leuconostoc. Of the Pediococcus are primarily P. acidilacti, P. pentosaceus, P. urinae, and of the Lactobacillus; L. acidophilus, L. jenseni, L. casei, L. fermentum; and of the Leuconostoc: Les. mesenteroides.

The isolation process follows known routine processes for the isolation of pure cultures. The isolated pure cultures are then typed according to known methods, e.g. API. The choice of suitable organisms is then made with regard to the desired properties, i.e. lactic acid production, and antagonistic properties against uropathogens. Thereby a choice of uropathogens representing uropathogen E. coli, Enterococcus but also those others actual pathogens belonging to staphylococcus, gonorrea, Chlamydia , Trichosomas, Gardnerella, Bacterioides, Mobuluncus, Ureaplasma, Urealyticum, Klebsiella, Candida, and others known in connection with UTI are tested.

The thus selected lactic acid producing bacteria are then cultivated in a fermentor in a manner known per se, are separated from the medium using a separator or a centrifuge, are freeze-dried in a manner known per se, and ground to a fine powder. The powderous bacterial concentrate thus obtained is then mixed with a fermentable carbohydrate e.g. glucose, so that the final concentration of about 100 billion viable lactic acid producing bacteria per gram are obtained. This bacterial powder is then used to be added to a tampon or sanitary napkin in the above given way so that each tampon or sanitary napkin then contains about 100 mg powder, corresponding to >10 billion viable lactic acid producing bacteria.

The addition of the bacterial powder on a tampon or sanitary napkin can suitably be carried out in two ways. According to one method the tampon or sanitary napkin is coated with an adhesive according to above, and then a relevant amount of powder is then blown onto or spread over the coated place. The amount relationship between the main organism Pediococcus and other complementary lactic acid producing bacteria is of subordinated significance, however, at least 50% of the total amount of viable lactic acid producing bacteria should consist of pediococcus. Thereby the bacterial powder is attached to the basic layer. Then the tampon or sanitary napkin is provided with the above mentioned permeable layer of a suitable material in order to thus protect the bacterial layer against mechanical damage. It is also convenient to pack the tampon or sanitary napkin in a protecting film of tight paper, polymer film, aluminium foil or multiple layer foil. According to the second method the desired amount of bacterial powder is applied in the form of a suspension wherein the bacterial powder is finely distributed in the adhesive agent to a homogeneous suspension. The application can take place e.g. by coating of the suspension or by dipping the tampon in the suspension. In some cases it is also possible to add the living bacteria to a tampon or sanitary napkin and then carry out a freeze drying thereof.

The tampon or sanitary napkin thus prepared has turned out to obstruct UTI, alleviate the troubles at acute and chronic UTI and to assist in a rapid recovery of the natural micro-organism flora of lactic acid producing bacteria after a antibiotic treatment.

A group of women which are in particular sensitive to UTI are the pregnant women. At UTI in pregnant women it is thus not advisable to treat the infection using an antibiotic. Then one should use a tampon or sanitary napkin of the present invention in stead when such infections are at hand.

## Claims

1. Tampon or sanitary napkin to be used prophylactically against urogenital infections, as well as at acute or chronic urogenital infections in order to alleviate the effects of the infections, and contribute to a rapid recovery of the natural flora of micro-organisms of the urogenital region after an antibiotic treatment, **characterized** in that the tampon or sanitary napkin is provided with a culture of at least one lactic acid producing bacteria comprising the genus Pediococcus, preferably isolated from the urogenital region of healthy individuals.

2. Tampon or sanitary napkin according to claim 1, **characterized** in that the bacterial culture is retained onto the tampon or sanitary napkin using an adhesive.

3. Tampon or sanitary napkin according to claim 1 to 2, **characterized** in that the bacterial culture added optionally contains a combination of other lactic acid producing bacteria, such as Lactobacillus and Leuconostoc.

4. Tampon or sanitary napkin according to claim 1, **characterized** in that the active bacterial culture consist of P. acidilacti, P. pentosaceus, P. urinae, optionally in combination with one or more of the Lactobacillus, preferably L. acidophilus, L. jenseni, L. casei, L. fermentum; or of the Leuconostoc, preferably Les. mesenteroides.

5. Tampon or sanitary napkin according to claim 2, **characterized** in that the adhesive consist of coco fat, vegetabilic oil, other glyceride or wax.

6. Tampon or sanitary napkin according to claims 1 to 2, **characterized** in that the addition of lactic acid producing bacterial culture takes place in the form of a suspension consisting of an adhesive and lactic acid producing bacteria.

7. Tampon or sanitary napkin according to any of the preceeding claims, **characterized** in that the bacterial layer is coated with a permeable layer of cellulose fiber for protecting purposes.

8. Tampon or sanitary napkin according to claims 1 to 7, **characterized** in that it is provided with a non-permeable protecting layer of polymer or other foil applied above the permeable layer.

9. Tampon or sanitary napkin according to claim 3, **characterized** in that the amount of bacteria added is at least 10⁴ and at most 50 x 10⁹ per unit.

## Patentansprüche

1. Tampon oder Binde zur Verwendung in der Prophylaxe von Urogenitalinfektionen sowie bei akuten oder chronischen Urogenitalinfektionen, um die Auswirkungen der Entzündungen zu mildern, und zur Unterstützung einer raschen Wiederherstellung der natürlichen Flora von Mikroorganismen im Urogenitalbereich nach einer Behandlung mit Antibiotika, dadurch gekennzeichnet, daß der Tampon oder die Binde mit einer Kultur zumindest eines milchsäureproduzierenden Bakteriums versehen ist, die die Gattung Pediococcus, die vorzugsweise aus dem Urogenitalbereich gesunder Personen entnommen wurde, umfaßt.

2. Tampon oder Binde nach Anspruch 1, dadurch gekennzeichnet, daß die Bakterienkultur mit Hilfe eines Haftmittels auf dem Tampon oder der Binde gehalten wird.

3. Tampon oder Binde nach Anspruch 1 oder 2, dadurch gekennzeichnet, daß die Bakterienkultur optional zusätzlich eine Kombination anderer milchsäureproduzierender Bakterien, wie z. B. Lactobacillus und Leuconostoc, enthält.

4. Tampon oder Binde nach Anspruch 1, dadurch gekennzeichnet, daß die aktive Bakterienkultur aus P. acidilacti, P. pentosaceus, P. urinae besteht, optional in Kombination mit einer oder mehreren Art(en) der Gattung Lactobacillus, vorzugsweise L. acidophilus, L. jenseni, L. casei, L. fermentum, oder der Gattung Leuconostoc, vorzugsweise Les. mesenteroides.

5. Tampon oder Binde nach Anspruch 2, dadurch gekennzeichnet, daß das Haftmittel aus Kokosfett, pflanzlichem Öl, einem anderen Glyzerid oder Wachs besteht.

6. Tampon oder Binde nach Anspruch 1 oder 2, dadurch gekennzeichnet, daß das Hinzufügen der milchsäureproduzierenden Bakterienkultur in Form einer Suspension erfolgt, die aus einem Haftmittel und milchsäureproduzierenden Bakterien besteht.

7. Tampon oder Binde nach einem der vorhergehenden Ansprüche, dadurch gekennzeichnet, daß die Bakterienschicht, zum Zweck ihres Schutzes, mit einer durchlässigen Schicht aus Zellulosefasern bedeckt ist.

8. Tampon oder Binde nach den Ansprüchen 1 bis 7, dadurch gekennzeichnet, daß er/sie mit einer undurchlässigen Schutzschicht aus einer Polymer- oder anderen Folie versehen ist, die sich über der durchlässigen Schicht befindet.

9. Tampon oder Binde nach Anspruch 3, dadurch gekennzeichnet, daß die Menge der zugefügten Bakterien mindestens 10⁴ und höchstens 50 x 10⁹ Einheit beträgt.

## Revendications

1. Tampon ou serviette sanitaire s'utilisant de façon prophylactique contre les infections urogénitales, de type aigu ou chronique, afin d'atténuer les effets des infections et de contribuer à rétablir rapidement la flore naturelle de micro-organismes de la région urogénitale après un traitement antibiotique, **caractérisé en ce** que le tampon ou la serviette sanitaire sont pourvus d'une culture de bactéries produisant au moins un acide lactique et comprenant le genre Pediococcus, de préférence isolé de la région urogénitale d'individus sains.

2. Tampon ou serviette sanitaire suivant la revendication 1, **caractérisé en ce** que la culture bactérienne est retenue contre le tampon ou la serviette sanitaire en utilisant un adhésif.

3. Tampon ou serviette sanitaire suivant la revendication 1 ou 2, **caractérisé en ce** que la culture bactérienne ajoutée, en option, contient une combinaison d'autres bactéries produisant un acide lactique, tel que Lactobacillus et Leuconostoc.

4. Tampon ou serviette sanitaire suivant la revendication 1, **caractérisé en ce** que la culture bactérienne active consiste en P. acidilacti, P. pentosaceus, P. urinae, éventuellement en combinaison avec un ou plusieurs des Lactoacillus, de préférence L. acidophilus, L. jenseni, L. casei, L. fermentum ; ou de Leuconostoc, de préférence Les. mesenteroides.

5. Tampon ou serviette sanitaire suivant la revendication 2, **caractérisé en ce** que l'adhésif consiste en graisse de coco, huile végétale, d'autres glycérides ou de cire.

6. Tampon ou serviette sanitaire suivant la revendication 1 ou 2, **caractérisé en ce** que l'addition de culture bactérienne produisant de l'acide lactique prend place sous la forme d'une suspension consistant en un adhésif et des bactéries produisant l'acide lactique.

7. Tampon ou serviette sanitaire suivant l'une quelconque des revendications précédentes, **caractérisé en ce** que la couche bactérienne est revêtue d'une couche perméable de fibres de cellulose pour des raisons de protection.

8. Tampon ou serviette sanitaire suivant l'une quelconque des revendications 1 à 7, **caractérisé en ce** qu'il est pourvu d'une couche protectrice non-perméable de polymère ou d'une autre feuille par dessus la couche perméable.

9. Tampon ou serviette sanitaire suivant la revendication 3, **caractérisé en ce** que la quantité de bactéries ajoutée est d'au moins 10⁴ et d'au plus 50 x 10⁹ par unité.
